# EUROPÄISCHE PATENTANMELDUNG

(11) **EP 1 157 686 A2**
(43) Veröffentlichungstag der Anmeldung: **28.11.2001**
(21) Anmeldenummer: 01111889.0
(22) Anmeldetag: 17.05.2001
(51) Int. Cl.: A61K 7/32

(54) **Kombinationen von Glycerinmonoalkylethern und Fettsäureglyceriden**

(30) Priorität: 20.05.2000 DE 10025122
(71) Anmelder: Beiersdorf Aktiengesellschaft, 20245 Hamburg (DE)
(72) Erfinder: Schreiber, Jörg, Dr., 22087 Hamburg (DE); Teichmann, Stephan, Dr., 22880 Wedel (DE); Wolf, Florian, Dr., 37671 Höxter (DE)

(57) **Zusammenfassung**

Gegenstand der Erfindung sind Zubereitungen, insbesondere kosmetische oder dermatologische, insbesondere topische Zubereitungen mit einem Gehalt an einer Kombination von
(A) einem Wirkstoff oder mehreren Wirkstoffen, ausgewählt aus der Gruppe der Glycerinmonoalkylether der Formel

   R-O-CH₂-CHOH-CH₂OR',

   und/oder 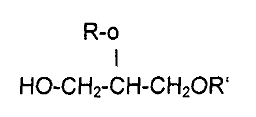 worin R eine geradkettige oder verzweigtkettige Alkylgruppe mit 3-24 Kohlenstoffatomen und R' H, -CH₂-CHOH-CH₂OH oder -CH₂-CHOH-CH₂-O-CH₂-CHOH-CH₂OH bedeutet, kombiniert mit
(B) einem Wirkstoff oder mehreren Wirkstoffen, ausgewählt aus der Gruppe der Fettsäuremonoglyceride und/oder Fettsäureoligoglyceride des Glycerins oder der Polyglycerine.

## Beschreibung

Gegenstand der Erfindung sind Zubereitungen, insbesondere kosmetische oder dermatologische, insbesondere topische Zubereitungen mit einem Gehalt an einer Kombination von einem Wirkstoff oder mehreren Wirkstoffen, ausgewählt aus der Gruppe der Glycerinmonoalkylether, kombiniert mit einem Wirkstoff oder mehreren Wirkstoffen, ausgewählt aus der Gruppe der Fettsäureglyceride.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Organismen sind in ihrer Eigenschaft als pathogene Keime von medizinischer Bedeutung, andere wiederum sind vollkommen unschädlich.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist das Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.
Eine besondere Aufgabe der vorliegenden Erfindung war es also, einen gegen unreine Haut bzw. Propionibacterium acnes wirksamen Wirkstoff zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [mukh 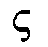 = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch eine Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind kohlenstoff-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ferner sind Superinfektionen der Haut durch Pilze und Bakterien nicht selten.

Bei bestehendem Primärinfekt, d.h. der normalen Keimbesiedelung der Haut, eintretender Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüsse eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Protozoen sind parasitisch lebende Einzeller mit klar abgegrenztem Zellkern, die sich ungeschlechtlich fortpflanzen (durch Teilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozoiten) unter ungünstigen Umständen auch Zysten als Dauerformen ausbilden,

Je nach Fortbewegungsart und -apparat werden Protozoen in vier verschiedene Gruppen unterteilt:
(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweie durch Leishmania donovanii, L.tropica, L.brasiliensis, L.mexicana, L.diffusa oder L. pifanoi), Trypanosmiasis (verursacht durch verschiedene Trypanosoma-Arten), Amöbenruhr und Amöbiasis (verursacht beispielsweise durch verschiedene Entamoeba-Arten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erheblich, das menschliche Wohlbefinden. Es besteht daher bei den betroffenen Personen ein erheblicher Bedarf, diesem Zustande abzuhelfen. Eine Aufgabe der vorliegenden Erfindung war es also, gegen Protozoen wirksame Wirkprinzipien zu finden.

Parasiten sind ein- oder mehrzellige Pflanzen oder Tiere, die sich auf (= Ektoparasiten) oder in (= Endoparasiten) anderen Lebewesen auf deren kosten ernähren, und zwar mit (= Pathogene Parasiten) oder ohne (Apathogene Parasiten) Verursachung von Krankheitserscheinungen. Die Lebensweise ist entweder auch aprophytisch oder aber rein parasitär, eventuell nur als periodischer, temporärer oder stationärer Parasit. Die Entwicklung von Parasiten ist an einen oder mehrere verschiedene Wirtsorgansimen gebunden, wobei der Mensch Zwischenwirt oder Endwirt sein kann.

Medizinisch und dermatologisch bedeutsame Parasiten sind beispielsweise die Helminthen, die sich wiederum in Trematodae, Cestodae und Nematodae untergliedern. Das menschliche Wohlbefinden beeinträchtigende Helminthosen sind beispielsweise Bilharziose, (verursacht durch Schistosoma-Arten), Bandwurmbefall vom Darm und anderen inneren Organen (verursacht durch beispielsweise Taenia-Arten und Echinococcus-Arten), Ascariasis (verursacht durch Ascaris lumbricoides), Enterobiasis (verursacht durch Enterobium vermicularis), Paragonimiasis (verursacht durch Paragonium-Arten), Filariose (verursacht beispielsweise durch Wucheria bancrofti) sowie anderer Nematodenbefall (beispielsweise verursacht durch Trichuris trichura oder Trichinella spiralis).

Darüber hinaus bestehen eine Vielzahl auf bzw. in Mensch und Tier parasitisch lebender Insektenarten bzw. Spinnentieren, die medizinische und dermatologische Veränderungen der Wirtsorganismen hervorrufen. In dieser Hinsicht für die Beeinträchtigung des menschlichen Wohlbefindens verantwortliche Parasitosen sind beispielsweise Accrodermatitis (verursacht durch Getreidemilben, beispielsweise Pediculoides ventricosus), Skabies (verursacht durch Sarcoptes scabii), Fliegen- und/oder Fliegenlarvenbefall (verursacht beispielsweise durch Glossina-, Stomoxys-, Tabanus-, Chrysops-, Lucilia-, Chrysomya-, Cochliamya-, Wohlfartia-, Cordylobia- oder Dermatobia-Arten), Mücken- und/oder Mückenlarvenbefall (verursacht beispielsweise durch Aedes- Culex-, Anopheles-, Phlebotomus- Culicuides-, Sumilium- oder Haemagoges-Arten), Zeckenbefall (verursacht beispielsweise durch Argas persicus und andere Argas-Arten, Ornithodorus erraticus und andere Ornithodorus-Arten, Orobius-Rhiphocephalus-, Dermacentor-, Haemaphysalis-, Amblyomma-, Ixodes-Arten), Porocephalose (verursacht durch Porocephalus-Arten), Flohbefall (verursacht durch beispielsweise Pulex irritans, Ctenocephalides canis, Xenopsylla cheopsis, Nosophyllus fasciatus oder Sarcopsylla penetrans), Läusebefall (verursacht beispielsweise durch Phthirius pubis, Pediculosus humanus oder Pediculosus captits), Wanzenbefall (verursacht beispielsweise durch Cimex lectularius, Cimex hemipterus, Panstrongylus megistus, Rhodnius prolixus, Triatoma dimidata, Triatoma infestans, Triatoma sordida oder Triatoma brasiliensis) sowie Milbenbefall (verursacht beispielsweise durch Demodex folliculorum und andere Demodex-Arten sowie durch Dermamyskus-Arten Glyciphagus domesticus, Pyemotes-Arten, Sarcoptes-Arten oder Trombicula-Arten).

Dabei ist von zusätzlicher Bedeutung, daß die auf oder im menschlichen Organismus lebenden Parsiten ihrerseits wieder Überträger von Bakterien, Mycota, Protozoen und Viren sein können, die Gesundheit und Wohlbefinden des Wirtsorganismus, beispielsweise des Menschen, nachhaltig beeinträchtigen können. Es bestand daher der Bedarf, gegen Parasitosen wirksame Wirkprinzipien zu finden, welche das medizinische oder dermatologische Erscheinungsbild zu verbessern imstande sind. Diesen Bedarf zu stillen, war daher eine weitere Aufgabe der vorliegenden Erfindung.

Im Gegensatze zu den prokaryotischen und eukaryotischen zellulären Organismen sind Viren [virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über einen eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen, die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesen Nachteilen abzuhelfen, also Substanzen zu finden, welche wirksam einen ein- oder mehrzelligen Organismus vor schädlichen Folgen einer Virusinfektion, sei es prophylaktisch oder therapeutisch, schützen.

Die erfindungsgemäßen Wirkstoffkombinationen und Zubereitungen damit können prophylaktisch verwendet werden und bewirken, daß sich nur noch geringen und keine störenden Ansammlungen von Mikroorganismen, Viren, Parasiten und Protozoen ausbilden, oder sie beseitigen auch bereits vorhandene Mikroorganismen, Viren, Parasiten und Protozoen und verringern so deren Anzahl.

Alle vorstehend und nachstehend genannten Aufgaben werden erfindungsgemäß gelöst. Die erfindungsgemäßen Wirkstoffkombinationen haben die erfindungsgemäßen, genannten Wirkungen auf Mikroorganismen, Viren, Parasiten und Protozoen und sind zur Behandlung der genannten Störungen und Krankheiten geeignet.

Mikroorganismen der Haut und die durch diese Organismen hervorgerufenen kosmetischen, dermatologischen und medizinischen Phänomene (z.B. Achselgeruch, Fußgeruch, Körpergeruch, Kopfschuppen, Akne, Superinfektionen bei atopischem Ekzem, Psoriasis oder allgemein schlechtem Immunzustand, aber auch Wundinfektionen) werden bislang im wesentlichen durch Anwendung von mehr oder weniger breitbandig wirkenden Mikrobiziden bekämpft. Die Nachteile der antimikrobiellen Therapie sind dabei die größtenteils mangelhafte Selektivität der eingesetzten Wirksysteme, Nebenreaktionen, Unverträglichkeiten und insbesondere die Ausbildung von Multiresistenzen.

Aufgabe der Erfindung war es z.B., die genannten Nachteile des Standes der Technik zu vermeiden und mildere, hautfreundlichere Produkte zur Bekämpfung von Bakterien, Mykota, Viren, Parasiten und Protozoen zu schaffen.

Alle genannten Aufgaben wurden erfindungsgemäß gelöst und die genannten oder angestrebten Wirkungen wurden erhalten.

Gegenstand der Erfindung sind Zubereitungen, insbesondere kosmetische oder dermatologische, insbesondere topische Zubereitungen mit einem Gehalt an einer Kombination von
(A) einem Wirkstoff oder mehreren Wirkstoffen, ausgewählt aus der Gruppe der Glycerinmonoalkylether der Formel

   R-O-CH₂-CHOH-CH₂OR',

   und/oder 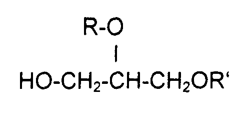 worin R eine geradkettige oder verzweigtkettige Alkylgruppe mit 3-24 Kohlenstoffatomen und R' H, -CH₂-CHOH-CH₂OH oder -CH₂-CHOH-CH₂-O-CH₂-CHOH-CH₂OH bedeutet, kombiniert mit
(B) einem Wirkstoff oder mehreren Wirkstoffen, ausgewählt aus der Gruppe der Fettsäuremonoglyceride und/oder Fettsäureoligoglyceride des Glycerins oder der Polyglycerine.

Geeignete Fettsäuren oder Monocarbonsäuren bzw. Fettsäurereste oder Acylgruppen (Alkylcarbonylgruppen) besitzen vorzugsweise 2-24 Kohlenstoffatome. Die Alkylreste können geradkettig oder verzweigtkettig sein. Es können Mono-, Di- oder Triglyceride mit den gleichen oder aber unterschiedlichen Fettsäureresten vorliegen.

Als Glyceride können die Glyceride des (Mono-)Glycerins oder die der Polyglycerine vorliegen, beispielsweise die des Diglycerins, Triglycerins oder Tetraglycerins, wobei auch gegebenenfalls diese weiteren Glycerinreste wie beschrieben O-acyliert sein können.
Weitere erfindungsgemäße, bevorzugte Monoglycerinmonoester werden durch die allgemeine Formel 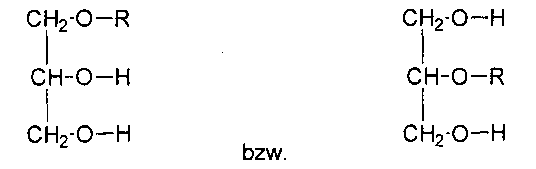 wiedergegeben, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die

| | | |
|---|---|---|
| Hexansäure | (Capronsäure) | (R= -C₅H₁₁), |
| Heptansäure | (Önanthsäure) | (R= -C₆H₁₃), |
| Octansäure | (Caprylsäure) | (R= -C₇H₁₅), |
| Nonansäure | (Pelargonsäure) | (R= -C₈H₁₇), |
| Decansäure | (Caprinsäure) | (R= -C₉H₁₉), |
| Undecansäure | | (R= -C₁₀H₂₁), |
| 10-Undecensäure | (Undecylensäure) | (R= -C₁₀H₁₉), |
| Dodecansäure | (Laurinsäure) | (R= -C₁₁H₂₃), |
| Tridecansäure | | (R= -C₁₂H₂₅), |
| Tetradecansäure | (Myristinsäure) | (R= -C₁₃H₂₇), |
| Pentadecansäure | | (R= -C₁₄H₂₉), |
| Hexadecansäure | (Palmitinsäure) | (R= -C₁₅H₃₁), |
| Heptadecansäure | (Margarinsäure) | (R= -C₁₆H₃₃), |
| Octadecansäure | (Stearinsäure) | (R= -C₁₇H₃₅). |

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den dermatologischen Zubereitungen beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Erfindungsgemäß liegen die Di- bzw. Triglycerineinheiten der erfindungsgemäßen Diglycerinmonocarbonsäure-monoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester des Diglycerins sind bevorzugt durch folgende Struktur gekennzeichnet (Substitutionspositionen angegeben): 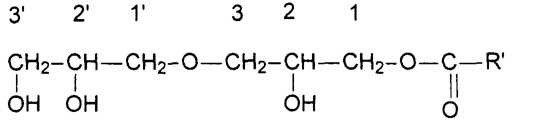 wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäuremonoester des Triglycerins sind bevorzugt durch folgende Struktur gekennzeichnet (Substitutionspositionen angegeben): 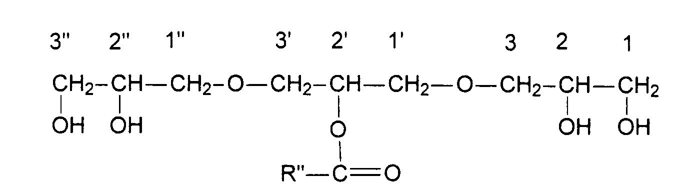 wobei R" einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Monocarbonsäuren sind die

| | | |
|---|---|---|
| Hexansäure | (Capronsäure) | (R' bzw. R"= -C₅H₁₁), |
| Heptansäure | (Önanthsäure) | (R' bzw. R"= -C₆H₁₃), |
| Octansäure | (Caprylsäure) | (R' bzw. R"= -C₇H₁₅), |
| Nonansäure | (Pelargonsäure) | (R' bzw. R"= -C₈H₁₇), |
| Decansäure | (Caprinsäure) | (R' bzw. R"= -C₉H₁₉), |
| Undecansäure | | (R' bzw. R"= -C₁₀H₂₁), |
| 10-Undecensäure | (Undecylensäure) | (R' bzw. R"= -C₁₀H₁₉), |
| Dodecansäure | (Laurinsäure) | (R' bzw. R"= -C₁₁H₂₃), |
| Tridecansäure | | (R' bzw. R"= -C₁₂H₂₅), |
| Tetradecansäure | (Myristinsäure) | (R' bzw. R"= -C₁₃H₂₇), |
| Pentadecansäure | | (R' bzw. R"= -C₁₄H₂₉), |
| Hexadecansäure | (Palmitinsäure) | (R' bzw. R"= -C₁₅H₃₁), |
| Heptadecansäure | (Margarinsäure) | (R' bzw. R"= -C₁₆H₃₃), |
| Octadecansäure | (Stearinsäure) | (R' bzw. R"= -C₁₇H₃₅). |

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Monocarbonsäuremonoester des Diglycerins denen des Triglycerins bevorzugt.

Ganz besonders günstig sind

| | | |
|---|---|---|
| Diglycerinmonocaprinat | (DMC) | R' = 9 |
| Triglycerinmonolaurat | (TML) | R" = 11 |
| Diglycerinmonolaurat | (DML) | R' = 11 |
| Triglycerinmonomyristat | (TMM) | R" = 13. |

Als bevorzugter erfindungsgemäßer Monocarbonsäuremonoester des Diglycerins hat sich das Diglycerinmonocaprinat (DMC) erwiesen.

Die erfindungsgemäßen Monocarbonsäuremonoester des Diglycerins liegen bevorzugt in 1-Stellung, die erfindungsgemäßen Monofettsäureester des Triglycerins bevorzugt in 2'-Stellung verestert vor.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Insbesondere vorteilhaft sind solche Monocarbonsäureester, welche nach einem Verfahren erhältlich sind, wie es in der DE-OS 38 18 293 beschrieben wird.

Die Diglycerinester, welche sich durch zwei, und die Triglycerinester, welche sich durch drei Asymmetriezentren auszeichnen, sind in all ihren Konfigurationen erfindungsgemäß wirksam. Die Diglycerinester besitzen vier, die Triglycerinester acht Stereoisomere.

Bei den Diglycerinestern sind die 2- und die 2'-Position Asymmetriezentren. Erfindungsgemäß aktiv und gleichermaßen von Vorteil sind die 2S2'S-, die 2R2'S-, die 2S2'R- und die 2R2'R-Konfiguration.

Bei den Triglycerinestern sind die 2-, die 2' und die 2"-Position Asymmetriezentren. Erfindungsgemäß aktiv und gleichermaßen von Vorteil sind die 2S2'S2"S-, die 2R2'S2"S-, die 2S2'R2"S-, die 2R2'R2"S-, die 2S2'S2"R, die 2R2'S2"R-, die 2S2'R2"R- und die 2R2'R2"R-Konfiguration.

Es hat sich als günstig herausgestellt, racemische Gemische der Stereoisomeren zu verwenden.

Gegenstand der Erfindung ist auch die Verwendung, insbesondere in Zubereitungen, insbesondere in kosmetischen oder dermatologischen, insbesondere topischen Zubereitungen, einer Kombination von
(A) einem Wirkstoff oder mehreren Wirkstoffen, ausgewählt aus der Gruppe der Glycerinmonoalkylether der Formel

   R-O-CH₂-CHOH-CH₂OR',

   und/oder 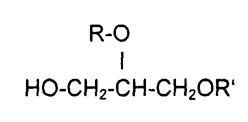 worin R eine geradkettige oder verzweigtkettige Alkylgruppe mit 3-24 Kohlenstoffatomen und R' H, -CH₂-CHOH-CH₂OH oder -CH₂-CHOH-CH₂-O-CH₂-CHOH-CH₂OH bedeutet,
   kombiniert mit
(B) einem Wirkstoff oder mehreren Wirkstoffen, ausgewählt aus der Gruppe der Fettsäuremonoglyceride und/oder Fettsäureoligoglyceride des Glycerins oder der Polyglycerine,

zur Bekämpfung von Bakterien, Mykota, Viren, Parasiten und Protozoen und zu der Behandlung der mit dem insbesondere kutanen Auftreten dieser Mikroorganismen verbundenen kosmetischen, dermatologischen und medizinischen Phänomene, z.B. Achselgeruch, Körpergeruch, Fußgeruch, Kopfgeruch, Akne, seborrhoische Dermatitis, mikrobielle Superinfektionen bei atopischem Ekzem, Psoriasis und Immunsuppression, sowie zur Konservierung oder bei Wundinfektionen.

Die Verwendung zur Konservierung oder als Konservierungsmittel betrifft insbesondere Kosmetika, Dermatika und Lebensmittel.

Gegenstand der Erfindung ist auch die Kombination der Wirkstoffe von (A) und (B).

Erfindungsgemäß bevorzugte Verbindungen (A) sind beispielsweise die folgenden Verbindungen: Hexoxyglycerin, Octoxyglycerin.

Besonders bevorzugte Kombinationen werden durch Kombinationen der vorstehenden Wirkstoffe (A) mit den Wirkstoffen (B) erhalten, wobei jeweils ein Wirkstoff oder mehrere Wirkstoffe eingesetzt werden können.

Bevorzugt werden die in den Beispielen genannten Wirkstoffe (A) oder (B). Bevorzugt werden auch Kombinationen der Wirkstoffe (A) und (B), die in den Beispielen genannt sind. Besonders bevorzugt werden Kombinationen der jeweils in einem Beispiel genannten Wirkstoffe (A) und (B),

Vorzugsweise beträgt die Menge der erfindungsgemäßen Verbindungen (B) in den Zubereitungen, insbesondere topischen Zubereitungen, 0,01 bis 20 Gew.-%, insbesondere 0,05 bis 5 Gew.-%, besonders bevorzugt 0,1 bis 3 Gew.-%, jeweils bezogen auf das Gesamtgewicht der Zubereitungen.

Vorzugsweise beträgt die Menge der erfindungsgemäßen Verbindungen (A) in den Zubereitungen, insbesondere topischen Zubereitungen, 0,01 bis 10 Gew.-%, insbesondere 0,05 bis 5 Gew.-%, besonders bevorzugt 0,1 bis 3 Gew.-%, jeweils bezogen auf das Gesamtgewicht der Zubereitungen.

Vorzugsweise werden die Glycerinether (A) in den Kombinationen mit den Verbindungen (B) in den jeweils angegebenen Gewichtsmengen verwendet. Das Verhältnis der Gewichtsmengen dieser Kombinations-Wirkstoffe A/B kann in den Zubereitungen stark variieren. Beispielsweise kann es 1/10 bis 10/1, oder 5/1 bis 1/5 betragen. Vorzugsweise kann es aber auch 1 /2 bis 2/1 und insbesondere 1/1 betragen.

Erfindungsgemäße Zubereitungen, die erfindungsgemäßen Wirkstoffkombinationen enthaltend, sind besonders vorteilhaft dadurch gekennzeichnet, dass die erfindungsgemäßen Wirkstoffkombinationen in Konzentrationen von 0,05 - 15,00 Gew.-%, bevorzugt 0,1 - 5,0 Gew.-% vorliegen, jeweils bezogen auf das Gesamtgewicht der Zubereitungen.

Auch die Verwendung der vorstehenden Stoffe und Kombinationen wird bevorzugt.

Die Aufbereitung der erfindungsgemäßen Wirkstoffe erfolgt nach den dem Fachmann geläufigen, üblichen Methoden. Erfindungsgemäße Wirkstoffe sind im Handel erhältlich oder bekannt oder können nach bekannten Verfahren erhalten werden.

Die Wirkstoffkombinationen sind gegenüber, insbesondere den vorstehend genannten, Mikroorganismen, Viren, Parasiten und Protozoen hervorragend wirksam und zur Behandlung der genannten Krankheiten und Zustände geeignet.

Die erfindungsgemäßen Wirkstoffkombinationen wirken insbesondere im Sinne von desinfizierenden Wirkstoffen, z.B. einer die Mikroorganismen, Viren, Parasiten und Protozoen abtötenden Wirkung oder z.B. in einer Wachstumshemmung für diese. Diese Wirkprinzipien werden insbesondere auch mit den Begriffen antimikrobielle, antivirale, antiparasitäre und gegenüber Protozoen antiparasitäre Wirkung gegenüber Mikroorganismen, Viren, Parasiten und Protozoen bezeichnet und sollen in gleicher Weise auch für diese Wirkstoffkombinationen gelten.

Es hat sich in erstaunlicher Weise herausgestellt, daß die erfindungsgemäßen Wirkstoffkombinationen das Wachstum von grampositiven und gramnegativen Bakterien, Mycobionten, Protozoen, Parasiten sowie Viren verhindern. Dabei wirken die erfindungsgemäßen Wirkstoffe in synergistischer Weise, also überadditiv in bezug auf die Einzelkomponenten (A) oder (B).
Insbesondere sind die erfindungsgemäßen Wirkstoffkombinationen befähigt, das Wachstum von Hefen, insbesondere der Pityrosporum-Arten, namentlich Pityrosporum ovale, zu verhindern.

Es hat sich ferner herausgestellt, daß die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, beseitigen.

Die erfindungsgemäßen Wirkstoffkombinationen eignen sich darüberhinaus gut für die Verwendung als desodorierender Wirkstoff in kosmetischen Desodorantien sowie gegen unreine Haut, leichte Formen der Akne bzw. Propionibakterium acnes.

Schließlich hat sich herausgestellt, daß die erfindungsgemäßen Wirkstoffkombinationen den Verderb organischer Substanz, insbesondere kosmetischer und dermatologischer Zubereitungen, durch den Befall mit grampositiven und gramnegativen Bakterien, Mycobionten, Protozoen, Parasiten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäßen Wirkstoffkombinationen gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten die erfindungsgemäßen Wirkstoffkombinationen in wirksamer Menge zugegeben werden.

Der Stand der Technik lieferte folglich nicht den geringsten Hinweis auf die erfindungsgemäße Verwendung als antimycotisches Wirkprinzip.

Ferner war erstaunlich, daß die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Anti-Schuppen-Schampoos.

Die Anwendung der Wirkstoffkombinationen kann topisch, perkutan, transdermal, parenteral, oral oder auch intravasal erfolgen.

Zubereitungen, die erfindungsgemäße Wirkstoffkombinationen enthalten, können topische Zubereitungen sein, beispielsweise kosmetische und dermatologische topische Zubereitungen oder aber auch übliche Arzneimittel-Darreichungsformen. Bevorzugt werden Desodorantien oder desodorierende Körperreinigungsprodukte oder Körperpflegeprodukte. Die Wirkstoffkombinationen können aber auch in Desinfektionsmitteln und/oder Reinigungsmitteln enthalten sein, die nicht nur zur behandlung des Körpers oder der Haut bestimmt sind, sondern auch zum Reinigen und Desinfizieren von harten Oberflächen, medizinischen Materialien, Geräten, Instrumenten, Mobiliar und Wänden.

Für den Körper bestimmte Reinigungsmittel, Desinfektionsmittel und Spülmittel können ebenfalls zur Behandlung der Haut verwendet werden, wie schon die topischen Zubereitungen. Sie dienen aber vorzugsweise zur Behandlung von Körperhöhlen, Wunden und auch des Mund- und Rachenraumes sowie der Nase.

Die erfindungsgemäßen Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, die erfindungsgemäßen Wirkstoffkombinationen mit anderen Wirkstoffen zu kombinieren, beispielsweise mit anderen antimikrobiell, antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen,
- Öle, wie Triglyceride der Caprin- oder der Caprylsäure, vorzugsweise aber Rizinusöl,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren,
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die erfindungsgemäßen Zubereitungen können auch Antioxidantien enthalten.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10,0 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwassertoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen. Die UVB-Filter können öl-löslich oder wasserlöslich sein. Als öl-lösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethyl0-hexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, erfindungsgemäße Wirkstoffe und Hilfsmittel, wie sie üblicherweise dafür verwendet werden.

Beispiele für oberflächen aktive Substanzen, die erfindungsgemäß vorteilhaft verwendet werden können, sind herkömmliche Seifen, z.B. Fettsäuresalze des Natriums, Alkylsulfate, Alkylethersulfate, Alkan- und Alkylbenzolsulfonate, Sulfoacetate , Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nichtionisches oder amphoteres Polymer oder auch Gemische davon, sowie erfindungsgemäße Wirkstoffe. Die Menge der verwendeten erfindungsgemäßen Wirkstoffkombinationen liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die die erfindungsgemäßen Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben erfindungsgemäßen Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Wirkstoffkombinationen in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Soweit nicht anders angegeben, beziehen sich Mengenangaben, Prozentangaben und Teile auf das Gewicht, insbesondere auf das Gesamtgewicht der jeweiligen Mischung oder Zubereitung.

Das folgende Beispiel soll die Verkörperungen der vorliegenden Erfindungen verdeutlichen. Die Angaben beziehen sich stets auf Gewichts-%, sofern nicht andere Angaben gemacht werden.

### Beispiel

| | **Gew.-%** |
|---|---|
| Propylenglycol | 3,22 |
| DMC | 1,00 |
| Octoxyglycerin | 1,00 |
| Octyldodecanol | 0,16 |
| Parfum | q.s. |
| Alkohol denat. | ad 100,00 |

### Beispiel 2

| **Aerosol Spray** | |
|---|---|
| Ethanol | 12,00 |
| DMC | 2,00 |
| Octoxyglycerin | 1,00 |
| Octyldodecanol | 2,00 |
| Ceterarylester | 2,00 |
| Alkohol denat. | 30,00 |
| Parfum | 2,00 |
| Treibgas | ad 100 |

### Beispiel 3

| **Deo Zerstäuber** | |
|---|---|
| PEG 40 hydrogenated Castor oil | 3.00 |
| DMC | 1.00 |
| Octoxyglycerin | 1.00 |
| Citrat-Puffer | 0.50 |
| Parfüm | 0.50 |
| Alkohol | 40.0 |
| Wasser | ad 100 |

### Beispiel 4

| **Roll-on** | |
|---|---|
| Hydroxyethylcellulose | 00.50 |
| Ethanol | 45.00 |
| Aluminum Chlorhydrate | 15.00 |
| Glycerylcaprinate | 01.00 |
| Octoxyglycerin | 01.00 |
| Parfüm | 01.00 |
| Wasser | ad 100 |

### Beispiel 5

| **Emulsions-Roll-on** | |
|---|---|
| PEG-40 Stearate | 05.00 |
| Cetylalkohol | 02.00 |
| Mineralöl | 02.00 |
| Polysorbate 80 | 01.00 |
| Glycerin | 01.50 |
| Mg-aluminum silicate | 00.80 |
| Aluminum Chlorhydrate | 15.00 |
| Triglycerinlaurate | 01.00 |
| Octoxyglycerin | 01.00 |
| Parfüm | 01.00 |
| Wasser | ad 100 |

### Beispiel 6

| **Roll-on** | |
|---|---|
| Steareth 2 | 02.00 |
| Steareth 21 | 05.00 |
| Ceterarylalkohol | 00.50 |
| Glycerylcaprylate | 01.00 |
| Octoxyglycerin | 01.00 |
| Parfüm | 01.00 |
| Wasser | ad 100 |

### Beispiel 7

| **AT-Aerosol** | |
|---|---|
| Silicone | 13.00 |
| Quaternium-18 hectorite | 00.80 |
| Ethanol | 00.80 |
| AT Puder | 10.00 |
| DMC | 01.00 |
| Octoxyglycerin | 01.00 |
| Parfüm | 01.00 |
| Wasser | ad 100 |

### Beispiel 8

| **Suspensions-Roll-on** | |
|---|---|
| Silicone | 63.00 |
| Quaternium-18 Hectorit | 13.50 |
| Silica | 00.50 |
| AT-Puder | 20.00 |
| DMC | 01.00 |
| Octoxyglycerin | 01.00 |
| Parfüm | 01.00 |

### Beispiel 9

| **Deo Tücher- Tränkmedium** | |
|---|---|
| PEG 40 hydrogenated Castor oil | 3.00 |
| Glycerylmonocaprylate | 1.00 |
| Octoxyglycerin | 1.00 |
| Citrat-Puffer | 0.50 |
| Parfüm | 0.50 |
| Alkohol | 40.0 |
| Wasser | ad 100 |

### Beispiel 10

| **Suspensionsstick** | |
|---|---|
| Stearylalkohol | 20.00 |
| Cyclomethicone | 54.00 |
| PPG-14 Butylether | 02.00 |
| Hydrogenated Castoroil | 01.00 |
| Talk | 02.00 |
| AT -Pulver | 20.00 |
| DMC | 1.00 |
| Octoxyglycerin | 1.00 |
| Parfüm | 0.50 |

### Beispiel 11

| **PIT-Emulsion** | |
|---|---|
| Glycerylstearate, Ceteareth-20, | |
| Ceteareth-10, Cetearyl alcohol, | |
| Cetyl Palmitate | 04.5 |
| Ceteareth 20 | 01.0 |
| Dicaprylylether | 05.0 |
| Dioctyl cyclohexan | 05.0 |
| DMC | 01.0 |
| Octoxyglycerin | 01.0 |
| Aluminum Chlorohydrate | 05.0 |
| Parfüm | 0.50 |
| Wasser | ad 100 |

### Beispiel 12

| **Deostift** | |
|---|---|
| Ethanol | 75.5 |
| DMC | 01.0 |
| Octoxyglycerin | 01.0 |
| Natriumstearate | 06.5 |
| Parfüm | 0.50 |
| Wasser | ad 100 |

### Beispiel 13

| **PIT-Emulsion** | |
|---|---|
| Glycerylstearate, Ceteareth-20, | |
| Ceteareth-10, Cetearyl alcohol, | |
| Cetyl Palmitate | 04.5 |
| Ceteareth 20 | 01.0 |
| Dicaprylylether | 05.0 |
| Dioctyl cyclohexan | 05.0 |
| DMC | 01.0 |
| Octoxyglycerin | 01.0 |
| Aluminum Chlorohydrate | 05.0 |
| Parfüm | 0.50 |
| Wasser | ad 100 |

### Beispiel 14

| **Mikroemulsion** | |
|---|---|
| DMC | 01.0 |
| Octoxyglycerin | 01.0 |
| Glycerinisostearate | 01.8 |
| Ceteareth 15 | 05.2 |
| Isotridecylisononanoate | 03.3 |
| Cyclomethicon | 06.6 |
| Parfüm | 0.50 |
| Wasser | ad 100 |

### Beispiel 15

| **Mikroemulsionsgel** | |
|---|---|
| Glycerylcaprinat | 01.0 |
| Octoxyglycerin | 01.0 |
| Glycerinisostearate | 01.8 |
| Aluminium Chlorohydrate | 05.0 |
| PEG-150 Distearate | 01.0 |
| Ceteareth 15 | 05.2 |
| Isotridecylisononanoate | 03.3 |
| Cyclomethicon | 06.6 |
| Parfüm | 0.50 |
| Wasser | ad 100 |

### Beispiel 16

| **AT-Tränkmedium für Tücher** | |
|---|---|
| Glycerylstearate, Ceteareth-20, Ceteareth-10, Cetearyl alcohol, Cetyl Palmitate | 04.5 |
| Ceteareth 20 | 01.0 |
| Dicaprylylether | 05.0 |
| Dioctyl cyclohexan | 05.0 |
| DMC | 01.0 |
| Octoxyglycerin | 01.0 |
| Aluminum Chlorohydrate | 10.0 |
| Parfüm | 0.50 |
| Wasser | ad 100 |

### Beispiel 17

| **DeoStift** | |
|---|---|
| Natriumstearate | 08.0 |
| Prpylenglycol | 10.0 |
| Parfüm | 0.50 |
| DMC | 01.0 |
| Octoxyglycerin | 01.0 |
| PPG-3 myristyl ether | 70.0 |
| Wasser | ad 100 |

## Patentansprüche

1. Zubereitungen, insbesondere kosmetische oder dermatologische, insbesondere topische Zubereitungen mit einem Gehalt an einer Kombination von
(A) einem Wirkstoff oder mehreren Wirkstoffen, ausgewählt aus der Gruppe der Glycerinmonoalkylether der Formel
R-O-CH₂-CHOH-CH₂OR',
und/oder 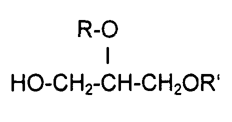 worin R eine geradkettige oder verzweigtkettige Alkylgruppe mit 3-24 Kohlenstoffatomen und R' H, -CH₂-CHOH-CH₂OH oder -CH₂-CHOH-CH₂-O-CH₂-CHOH-CH₂OH bedeutet,
kombiniert mit
(B) einem Wirkstoff oder mehreren Wirkstoffen, ausgewählt aus der Gruppe der Fettsäuremonoglyceride und/oder Fettsäureoligoglyceride des Glycerins oder der Polyglycerine.

2. Kombination von Wirkstoffen (A) und (B) gemäß Anspruch 1.

3. Verwendung, insbesondere in Zubereitungen, insbesondere in kosmetischen oder dermatologischen, insbesondere topischen Zubereitungen, einer Kombination von
(A) einem Wirkstoff oder mehreren Wirkstoffen, ausgewählt aus der Gruppe der Glycerinmonoalkylether der Formel
R-O-CH₂-CHOH-CH₂OR',
und/oder 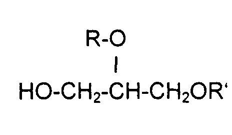 worin R eine geradkettige oder verzweigtkettige Alkylgruppe mit 3-24 Kohlenstoffatomen und R' H, -CH₂-CHOH-CH₂OH oder -CH₂-CHOH-CH₂-O-CH₂-CHOH-CH₂OH bedeutet,
kombiniert mit
(B) einem Wirkstoff oder mehreren Wirkstoffen, ausgewählt aus der Gruppe der Fettsäuremonoglyceride und/oder Fettsäureoligoglyceride des Glycerins oder der Polyglycerine,
zur Bekämpfung von Bakterien, Mykota, Viren, Parasiten und Protozoen und zu der Behandlung der mit dem insbesondere kutanen Auftreten dieser Mikroorganismen verbundenen kosmetischen, dermatologischen und medizinischen Phänomene, z.B. Achselgeruch, Körpergeruch, Fußgeruch, Kopfgeruch, Akne, seborrhoische Dermatitis, mikrobielle Superinfektionen bei atopischem Ekzem, Psoriasis und Immunsuppression, sowie zur Konservierung oder bei Wundinfektionen.

4. Zubereitungen gemäß den Ansprüchen 1, 3 und 4, **dadurch gekennzeichnet, daß** die Zubereitungen topische Zubereitungen sind.

5. Verwendung der Kombinationen oder Zubereitungen gemäß den Ansprüchen 1-5 als Desodorantien.
